# EUROPEAN PATENT APPLICATION

(11) **EP 2 390 002 A2**
(43) Date of publication of application: **30.11.2011**
(21) Application number: 10705621.0
(22) Date of filing: 13.01.2010
(51) Int. Cl.: B01J 35/00, B01D 53/86, C01B 33/36

(54) **PHOTOCATALYTIC MIXTURE FOR THE DEGRADATION OF NITROGEN OXIDES**

(30) Priority: 23.01.2009 ES 200900188
(71) Applicant: FMC Foret S.A., 08174 Sant Cugat del Vallés (Barcelona) (ES)
(72) Inventor: ARTIGAS PUERTO, Ramón, E-08174 San Cugat Del Valles (ES); GARCÍA GÓMEZ, Hermenegildo, E-08174 San Cugat Del Valles (ES); LLABRES XIMENA, Francesc, E-08174 San Cugat Del Valles (ES); DOMÍNGUEZ TORRES, Esther, E-08174 San Cugat Del Valles (ES)
(74) Representative: Durán Moya, Carlos
(86) International application number: PCT/ES2010/070014
(87) International publication number: WO 2010/084226

(57) **Abstract**

The invention relates to a photocatalytic mixture for the degradation of nitrogen oxides. A first aspect of the invention consists of a photocatalytic mixture intended for the degradation of nitrogen oxides through the synergic combination of two compounds: TiO₂ in any of the crystalline phases thereof or in the amorphous state, and one or more feldspars, such as carnegieite or nepheline. A second aspect of the invention consists in obtaining the aforementioned combination, thereby producing products capable of nitrogen oxide degradation.

## Description

### OBJECT OF THE INVENTION

A first aspect of the invention refers to a photocatalytic mixture designed to degrade nitrogen oxides through a synergic combination of two compounds: TiO₂, in any of its crystalline phases or in amorphous state, and one or various feldspars such as carnegeit or nepheline.

A second aspect of the invention refers to obtaining said combination giving rise to products with the capacity for degrading nitrogen oxides.

### BACKGROUND TO THE INVENTION

Feldspars are aluminosilicates comprising aluminium, silica and oxygen. Their structure consists of a silica base, part of which has been isomorphically replaced by aluminium. As they become unbalanced the charges are compensated with metal cations (K⁺, Na⁺, Ca⁺2). These solid materials are able to absorb compounds on their surface.

In turn, related materials exist known as feldspartoids which are a group of tectosilicates, similar to feldspartoids but having a different structure and a much lower silica content. The Si/Al ratio is practically 1:1 in feldspartoids however, it is close to 1:3 in feldspars.

In addition, feldspartoids have crystalline structures with pores and lower network density than feldspars. These pores are not interconnected and do not permit diffusion of ions or molecules in their interior.

The application of both feldspars and feldspartoid materials to alleviate the problem of atmospheric pollution caused by nitrogen (NOₓ) thus reducing the concentration of these gases in contaminated air presents the problem that, although the feldspars and feldspartoid materials have some capacity for adsorption, when they are saturated their capacity for reducing the percentage of NOₓ disappears. NOₓ is considered to be any nitrogen oxide irrespective of the atomic ratio N/O and its mixtures, in any proportion.

The maximum amount of NOₓ that the feldspar and the feldspartoid materials is able to absorb depends on the presence in the air of other substances, in addition to N₂, O₂ y H₂O, which may compete for adsorption of nitrogen oxides in the solid.

Thus, the maximum amount of NOₓ adsorbed is greater when the NOₓ is in a dry air stream than when it is in a damp air stream, as in the latter case adsorption of the NOₓ competes (unfavourably) with the adsorption of water in the damp air.

Therefore, the capacity for removing NOₓ from the air is not due to a degradation phenomenon of the NOₓ, but to absorption of the gas on the feldspar and feldspart^{x}oid material and, furthermore, this reaches a degree of saturation. In fact, it is necessary for the surface of the feldspar or mixture of feldspars to be free to achieve maximum NOₓ adsorption capacity, for NOₓ which in order to be used as adsorbent is required to activate the feldspars prior to use, for example, by means of a thermal treatment at 350°-450° to vacuum in N₂ stream.

In addition, the use of pure TiO₂ is also known for obtaining a reduction in the amount of NOₓ in the air provided that the material is irradiated with ultraviolet light.

In the case of TiO₂, the reduction of NOₓ concentrations in the air is not preferentially due to an adsorption phenomenon (as we have determined that the reduction of NOₓ concentration in a gaseous effluent in the absence of light is negligible) but rather due to the action of a photocatalytic process.

The elimination of NOₓ from the air in this case is certainly carried out through chemical transformation of the nitrogen oxide; however, experiments performed under laboratory conditions show that the activity of the TiO₂ is restricted, given that a drop in the titanium oxide activity occurs, until the final ending of said activity.

This invention consists of a photocatalytic mixture which combines in a synergic manner TiO₂ and feldspartoid materials, such as carnegeit and nepheline, in such a way that said photocatalytic mixture shows an activity of the individual components producing a synergy which determines that the activity continues rather than ending.

### DESCRIPTION OF THE INVENTION

A first aspect of the invention consists of a photocatalytic mixture for degrading nitrogen oxides This photocatalytic mixture comprises a combination of TiO₂ and carnegeit, or TiO₂ and mixtures of feldspartoids(carnegeit together with nepheline or both together with mullite in varying proportions) in such a way that a synergic relation is established between the TiO₂ and the feldspartoid materials. Feldspartoid materials are considered to be a group of tectosilicates which are similar to feldspartoids, however, they have a different structure and a much lower silica content. The Si/Al ratio is practically 3:1 in feldspartoids however, it is close to 1:1 in feldspars. In addition feldspartoids have crystalline structures with pores and less network density than feldspars. These pores are not interconnected and do not permit the diffusion of ions or molecules in their interior.

Feldspars have the general formula Xal₍₁₋₂₎ Si₍₃₋₂₎ O₈ where X in the formula may be Na and/or K and/or Ca. Generally, the Si/Al ratio in feldspars is 1:1. The atoms of Si and Al occupy the centres of tetrahedrons connected by their vertices forming a negatively charged three dimensional network. The cations X in the formula ensure the electro-neutrality of the solid.

The TiO₂ in the mixture maintains its photocatalytic activity in the presence of ultraviolet light in such a way that in contact with a gaseous environment with presence of NOₓ, it reacts with the NOₓ giving rise to other compounds and as a result the NOₓ is removed from said surrounding environment.

This behaviour, which comes to an end in the case of TiO₂ in its pure state, is extended for a considerably longer period in the case of the mixture based on a combination of TiO₂ and feldspartoid. Initially the NOₓ are removed from the surrounding environment in a slightly more intense manner than if the TiO₂ were pure; and after having been reduced it reaches a stationary and asyntotically constant state which is maintained for a considerably longer period than in the absence of feldspartoid.

As will be described in the detailed explanation of the invention, the manner in which the feldspartoid material and the TiO₂ are combined may differ according to various embodiments.

It is important from the perspective of performance of the photocatalytic mixture obtained, with respect to its NOₓ degradation activity, that there exists an intimate union of both components, for example, through molturation or compacting in order to ensure high particle contact.

The feldspartoid usually has a granulometry much greater than that of the TiO₂.

A second aspect of the invention establishes a procedure for obtaining the photocatalytic mixture by milling both components in such a way that the feldspartoid granulometry is reduced aligning it and establishing the desired degree of contact.

A third procedure for obtaining the photocatalytic mixture makes use of wet feldspartoid. Wet feldspartoid with a granulometry greater than TiO₂ powder is able to retain said TiO₂ on its surface preferably applied through pulverisation. In this way TiO₂ is deposited on the large sized particles of the feldspartoid. A subsequent drying process ensures the definitive binding of the two components.

Another mode of preparing the mixture containing feldspartoids and titanium oxide is based on solid feldspartoid precursor materials. These precursors suffer a transformation in the feldspartoid solids through any physical or chemical treatment. One procedure for carrying out the transformation to feldspartoid is by firing the precursor at temperatures exceeding 500°C for a period of over 30 minutes.

The combination with titanium oxide may be carried out prior to or subsequent to the transformation of the precursor into foldspartoid.

According to the aforementioned procedure, it would be possible to obtain the photocatalytic mixture of this invention if zeolite A and titanium oxide in anathase phase are combined and this mixture is thermally treated at 800°C for 90 minutes, thus complete transformation of the zeolite occurs in the carnegeit feldspartoid with a certain proportion of nepheline.

Titanium dioxide in turn is transformed into rutile, and does not produce any solid containing titanium and silica in its composition.

Feldspartoid, coated with TiO₂ mantains its interactive capacity with the TiO₂ in such a way that it has been proved that it continues to show the synergic effect of the two components.

It is considered that the embodiments of mixture described in claims 2 to 8 are incorporated by reference in this description.

The constructive elements described in claims 9 and 10 are considered to be incorporated in this description by reference.

The use of the photocatalytic mixture according to claim 11 is considered to be incorporated in this description by reference.

The procedures for obtaining this photocatalytic mixture according to claims 12 to 17 are considered to be incorporated in this description by reference.

### DESCRIPTION OF THE DRAWINGS

The present description is complemented by a set of drawings illustrating a preferred embodiment of the invention, but which is in no way restrictive.
Figure 1 shows a diagrammatic illustration of a photocatalytic mixture according to an embodiment of the invention comprising particles of feldspartoids and TiO₂ particles. The particles are compacted and have a similar granulometry.
Figure 2 shows a diagrammatic illustration of a particle recovered from a photocatalytic mixture according to an embodiment of the invention comprising a nucleus of carnegeit cotaed with TiO₂ particles.
Figure 3 is a graph which shows the development of the NOₓ concentration both in a dry air current and in a wet air current when only carnegeit is used for removing the NOₓ. The discontinuous curve represents the development in a damp air current. The continuous curve represents the development in a dry air current.
Figure 4 shows the temporary profile of the NOₓ concentration present in an saturated damp air current when it is passed through a tubular reactor illuminated with UV light through glass fibre and which contains a bed of TiO₂, as well as mixtures of TiO₂ and carnegeit in the following proportions 75/25, 50/50, 25/75 and 10/90.
Figure 5 is a graph showing the variation in concentration of NOₓ based on the time in an experiment according to ISO 22197-1:2007(E) standard for different weights of the photocatalytic mixture.

### DETAILED EXPLANATION OF THE INVENTION

The invention consists of a photocatalytic mixture designed to degrade nitrogen oxides when it is subjected to electromagnetic radiation, preferably within the spectrum of ultraviolet rays.

The photocatalytic mixture comprises TiO₂ and feldspartoids in which it has been verified that a synergic relation has been established between the two components. This synergic relation consists of ensuring that the TiO₂ activity is permanent.

When the photocatalytic mixture enters into contact with an air current containing NOx, and applying UV radiation, a photocatalytic mixture activity is observed which is substantially greater than when the TiO₂ is used in its pure state.

Following this first synergic effect associated with an increase in the activity of the photocatalytic mixture during a certain period of time, a second synergic effect is observed. When this initial period of time has passed, which is of the same order of magnitude as the period during which the TiO₂ in its pure state is active, it is verified that the photocatalytic mixture does not lose its activity but stabilises round an asyntotically constant value which is maintained for a period which is considerably longer than for a photocatalyser containing pure TiO₂.

Both synergic effects are relevant because the first enhances the degradation capacity of the NOₓ and the second permits the photocatalytic mixture to operate continuously and in a stationary manner for a considerably longer period than that which operates with pure titanium dioxide.

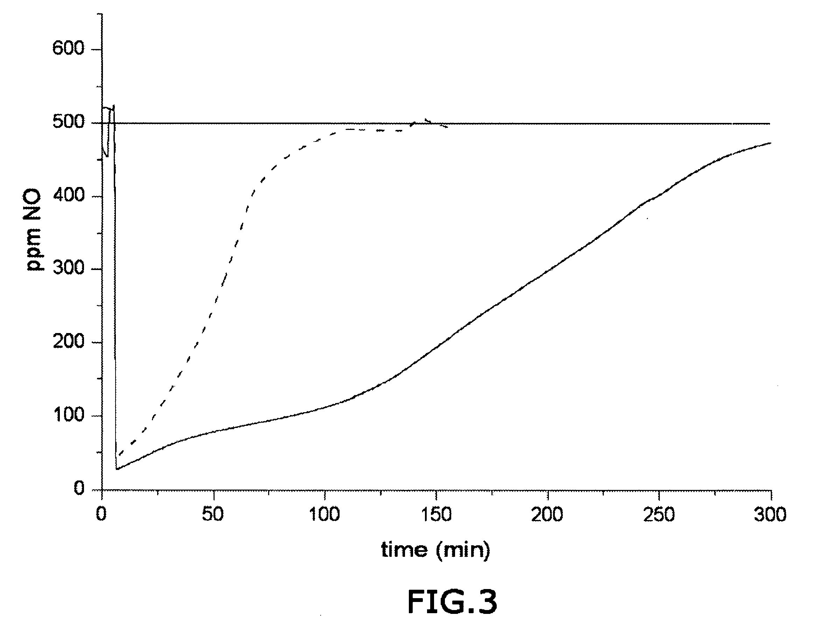

In order to highlight the synergic effect of the photocatalytic mixture compared with its components separately, figure 3 shows a graph obtained on the basis of data from an experiment consisting of checking the capacity for removing nitrogen oxides through the use of carnegeit with a certain amount of nepheline.

It has already been described how feldspartoids only have the capacity for removing nitrogen oxide from the air flow based on an effect of superficial adsorption.

The graph shows a first continuous curve well under the 500 marking which is the value for the concentration of NOₓ in the injected current. The variable of the abscisses is time. As time passes the function increases due to the fact that the feldspartoid material becomes saturated and its adsorption capacity is reduced.

In the experiment it is observed that after a lapse of 300 minutes the feldspartoid material is almost saturated and does not remove any further NOₓ from the air current.

The same graph shows another discontinuous curve. This curve represents the concentration of NOₓ when the air is damp. In this case, the water molecules in the damp air compete with the NOₓ molecules in order to occupy the surface of the feldspartoid material. The saturation of the feldspartoid material is produced much earlier and therefore the concentration of NOₓ reaches the value of the supply current much earlier.

In both cases there is no degradation of the NOₓ nor is there any permanent capacity for removing NOₓ from the air.

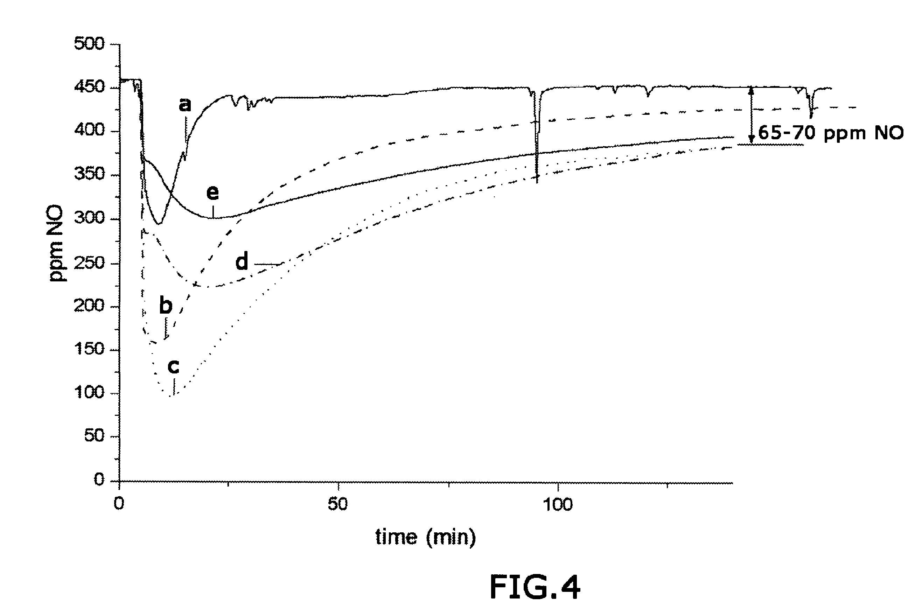

Figure 4 shows a graph which permits to observe both the behaviour of the TiO₂ and the photocatalytic mixture according to different modes of carrying out the invention.

The graph shows the values for the concentration of NOₓ based on the time in a current of air passing over a surface comprising different materials under photocatalytic conditions.

The graph shows curves identified with the following letters: a, b, c, d y e. Each of these curves corresponds to materials in the following table:

| | TiO₂/carnegeit mixtures |
|---|---|
| a. | 100 TiO₂ (% weight) |
| b. | 75 TiO₂ (% weight) |
| c. | 50 TiO₂ (% weight) |
| d. | 25 TiO₂ (% weight) |
| e. | 10 TiO₂ (% weight) |

Clearly the curve "a" corresponds to a photocatalytic mixture as it comprises solely TiO₂.

This curve "a" is that which serves as a reference for comparing the synergic effect of the combination of components in the invention.

The curve "a" has a degrading effect on the NOₓ for approximately 45 minutes. After this time the TiO₂ becomes saturated and is no longer able to act. The TiO₂ needs to be regenerated in order to have photocatalytic activity again.

As the TiO₂ is combined with feldspartoid material, an increase is observed in the capacity for elimination of NOₓ from the flow to which the material is subjected.

This increase is not due to the adsorption capacity of the feldspartoid material as it is negligible in a same experiment compared to the elimination capacity of TiO₂ through degradation.

It has been observed that the synergic effect is established in vary wide ranges of values such as that established between 10 and 90% in weight of TiO₂ with respect to the feldspartoid material. Nevertheless, it has been verified that for values between 40% and 60% the best results are obtained.

The experiment on the basis of which the graphs shown were obtained made use of a mixture of carnegeit and TiO₂ in anathase phase irradiated with a source of UV light. This light source may be either natural or artificial. While the mixture is irradiated an air current containing NOₓ is passed through it.

The result is a reduction in the amount of NOₓ in the outlet current due to a photodegradation phenomenon.

It was determined that the reduction of the amount of NOₓ in the current was due solely to photodegradation by repeating the experiment in the absence of radiation.

The effect of adsorption was determined as negligible compared to the effect of reduction when there is a UV radiation.

As may be seen in the graph, the amount of NOₓ degraded depends on the proportion of the two solids(TiO₂ and carnegeit) contained in the mixture, however in all the proportions studied (between a value lower than 100 and greater than 10% in weight of TiO₂) better results are always obtained using mixtures of TiO₂/carnegeit than when pure TiO₂ is used. Therefore, it may be stated that there is a synergic effect between carnegeit and TiO₂.

UV light was applied by using a 75W Xe lamp irradiating a surface of 15cm² containing lg of photocatalyser comprising a mixture of TiO₂ and carnegeit in the desired proportion.

The resulting conditions in the test are 5 W•cm⁻² which is approximately an order of magnitude greater than the maximum power of the sun on the surface of the earth.

Alternatively, in accordance with ISO 22197-1:2007(E) standard a series of three 8W "Blacklight blue lamps" were used which were placed parallel to the surface where the powder to be irradiated was deposited which is equivalent to approximately 64 times the strength of UV light of the sun.

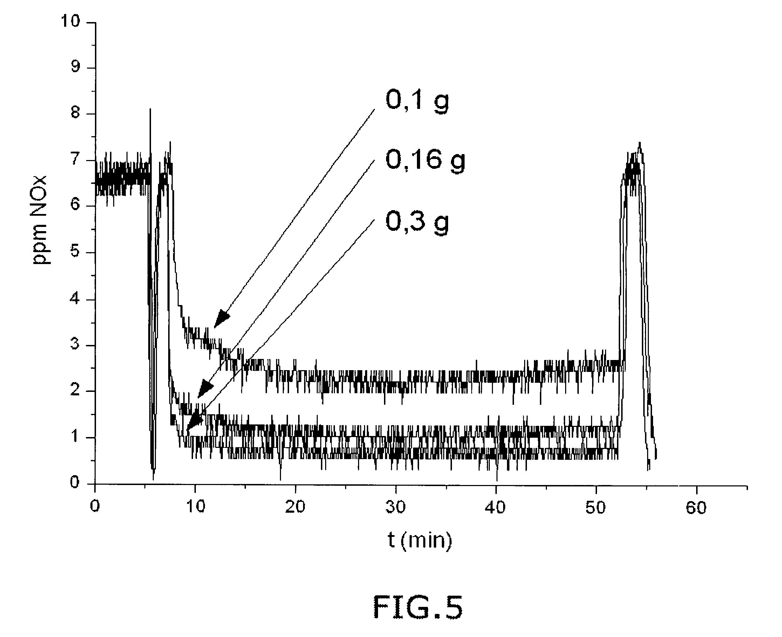

In the conditions laid down in the standard ISO 22197-1:2007(E) UV irradiation of the photocatalytic mixture was performed observing the reduction in the concentration of NOₓ shown in figure 5 based on the weight of the photocatalytic mixture.

Two synergic effects have already been described, the first in which the photodecomposition activity of NOₓ is greater during the first 45 minutes (in this example of an embodiment) and the second of maintenance of the activity over time.

During this last phase the mixture continues to be capable of decomposing the NOₓ in a continuous manner provided that the mixture is kept irradiated with UV light.

The stability tests carried out showed that this phase of stationary operation is maintained for at least 6 hours.

Having been apprised of its existence, the origin of this unexpected synergy was studied. The study consisted of washing the photocatalyser with aliquots of 20 ml of water and analysing the washing water using ionic chromatography.

This analytical technique unequivocally established the presence of nitrate and nitrite anions in a proportion of 98% to 2%.

It is known in the state of the art that nitrate and nitrite ions when deposited on the surface of titanium oxide cause the loss of photocatalytic activity.

Therefore, from the synergy between TiO₂ and feldspartoid materials it may be deduced that the anions produced from the photo oxidation of the nitrogen oxides pass preferably to being deposited on the surface of the feldespartoid material and therefore the TiO₂ remains unaltered in its photocatalytic activity while the feldsaprtoid material acts by trapping nitrate and nitrite ions.

As a result, the effect observed in the claimed photocatalytic mixture arises from an unexpected mechanism in which reactive species are generated by the action of light on the surface of the titanium dioxide, however when it reacts in gas phase with the nitrogen oxides the reaction products (nitrates and nitrites) which otherwise would act as TiO₂ poisons are preferably deposited in the feldspartoid material. The origin of this preferred absorption in the feldspartoid material may derive from the proximity of the TiO₂ particles and the feldspartoid material, together with the greater specific superficial area of the felspartoid material. This mechanism is not known in the chemical literature available and would serve as a base for explaining the unexpected origin of the synergy between both components of the photocatalytic mixture.

It has been experimentally proven that the regeneration of the combination takes place through simple washing with water. In the washing the ions which are produced by the photocatalytic oxidation dissolve in the water, being capable to recover the initial photocatalytic activity of the system if the washing completely eliminates the ions formed.

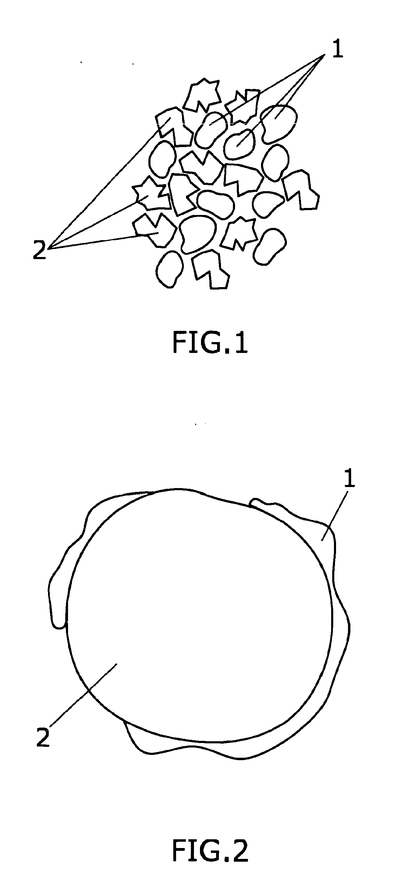

Figure 1 shows a diagrammatic illustration of an embodiment of the invention according to claim 2 in which a compacted mixture of TiO₂ (1) and the feldspartoid material (2) is represented.

The mixture in this example of an embodiment, derives from TiO₂ (1) powder and felspartoid material powder (2).

As the feldspartoid material powder (2) used in this example of an embodiment originally has a much greater granulometry, a milling stage was carried out in order to obtain the same granulometry for the two components. One mode of milling operation is to use a microsphere mill.

With this degree of compacting between the two components (1, 2) of the mixture there is a very high degree of contact between the particles which encourages the synergic effect between both components (1, 2).

Figure 2 shows a diagrammatic illustration of a particle pertaining to a mode of embodiment of the invention according to claim 3. The particle shows a nucleus formed by a particle of feldspartoid material (2) on which there is a coating of TiO₂ (1).

A mode of obtaining this structure consists of taking powder from the wet feldspartoid material (2) and TiO₂ powder (1) with a granulometry of the feldspartoid material (2) greater than that of the TiO₂ (1), and carrying out on the feldspartoid (2) a projection of TiO₂ powder (1), until the feldspartoid material (2) has been covered. Finally the mixture obtained is left to dry.

A mixture obtained according to any of the modes of embodiment of the invention may be used, applying it on a surface exposed to a gaseous environment containing nitrogen oxides. In order to ensure that the photocatalytic mixture is active, the surface on which the photocatalytic mixture has been applied is exposed to UV radiation of either artificial light or sunlight.

In addition this photocatalytic mixture may take the form of an additive in paints, ceramics or other construction materials which will be exposed to natural sunlight or artificial light.

In order to enhance application of the photocatalytic mixture on a surface, within the invention a photocatalytic mixture which additionally comprises a fixation substrate is considered. This fixation substrate may be a paint, a varnish; that is, a matrix in which the photocatalytic mixture is embedded.

This matrix should be such that it does not impede activity of the photocatalytic mixture on the surface when radiation acts on it.

A mode of embodiment which is of considerable practical interest is a construction element which comprises a substrate support of the block or plate type, in which either in its composition or at least on one of its faces it includes a layer or an impregnation of the photocatalytic mixture according to any of the modes of embodiment.

After having used this construction element to form part of a wall, a floor or a ceiling, a surface is generated (that which exposes the face which has a layer or impregnation of the photocatalytic mixture when this is not part of the formulation) which is able to degrade NOₓ gases in the surrounding environment, preferably air.

The manufacture of this type of construction elements has a significant disadvantage which has also been addressed in this invention. Manufacture of ceramics involves the photocatalytic mixture being subjected to a thermal treatment. If the ceramic incorporates a mixture such as that which is the subject of this invention, it may also be subject to changes due to the thermal treatment which affects its performance.

Therefore, when the manufacturing process of the construction element involves a firing stage it may be based on a mixture of titanium oxide and a precursor of the feldspartoid solids.

For example, it may be based on a mixture of titanium dioxide and 4A zeolite. In the firing process this mixture spontaneously changes depending on the time and temperature of the firing in order to give rise to a photocatalytic combination which constitutes one of the preferred embodiments of this invention.

Thus, when starting from anathase and 4A zeolite and it is used as a ceramic additive, by heating to 800°C for 90 minutes the initial mixture is transformed to rutile and carnegeit containing nepheline which is a photocatalytically active composition in order to decompose nitrogen oxides.

Heating to higher temperatures increases the percentage of nepheline to the detriment of the percentage of carnegeit with this mixture of feldspartoids also being active photocatalytically when it is present in the combination of titanium dioxide.

The feldspartoid finally obtained when the temperature is sufficiently high and the firing time is sufficient is mullite.

It has been observed that the photocatalytic mixture gradually reduces its photocatalytic activity as the firing temperature and the time progressively increase.

In accordance with the mechanism proposed in this invention, this gradual loss of photocatalytic activity may derive from the combination of a reduction in the surface area and porosity of the feldspartoid material, together with the transformation of titanium dioxide in phases of less photocatalytic activity. This means that if the mixture is incorporated in the ceramic and this is thermally treated, the result exhibits a photocatalytic activity in order to degrade the nitrogen oxides which are in contact with the ceramic which at least is a function of the temperature and time of firing.

By means of experiments, it was found that carnegeit and nephelines are feldspartoid materials which possess synergic character with TiO₂. These feldspartoid phases are stable up to temperatures below 1100°C, where carnegeit progressively transforms into nepheline and this in turn becomes mullite. Taking into account the influence of the temperature on photocatalytic activity of the combination containing feldspartoid material, mullite is the phase which exhibits the least efficacy.

At temperatures lower than 800°C both carnegeit and nepheline have been proved to remain stable, thus when the firing is finished there is no reversion of phases.

As an example of a preferred embodiment, the manufacture of a ceramic element through conventional means is proposed (which may be carried out at very high temperatures above 1100°C) in such a way that, the photocatalytic mixture resistant to temperature is applied after firing. The photocatalytic mixture may be applied by means of an enamel which is once more fired at a lower temperature, for example between 600°C and 800°C. The result is a fired ceramic element with an active surface for degradation of nitrogen oxides of surrounding gases.

## Claims

1. Photocatalytic mixture for degradation of nitrogen oxides **characterised in that** it comprises a photocatalytically active combination of TiO₂ and feldspartoid material.

2. Mixture according to claim 1 **characterised in that** the combination comprises a mixture of TiO₂ powder and feldspartoid material composed of carnegeit, nephelite or a combination of both in any proportion.

3. Mixture according to claim 1 **characterised in that** the combination comprises felspartoid material the particles of which are coated with TiO₂.

4. Mixture according to any of claims 1 to 3 **characterised in that** the TiO₂ is in anathase, rutile, amorphous phase or a mixture of these in any proproportion.

5. Mixture according to any of claims 1 to 3 **characterised in that** the feldspartoid material is carenegeit, nephelite, mullite or a combination of these in any proportion.

6. Mixture according to any of the previous claims **characterised in that** the proportion of TiO₂/feldspartoid material in weight is between 10% and 90% of weight of TiO₂ with respect to the weight of feldspartoid material.

7. Mixture according to claim 6 **characterised in that** the proportion of TiO₂/feldspartoid material in weight is between 40% and 60% of weight of TiO₂ with respect to the weight of feldspartoid material.

8. Mixture according to any of the previous claims **characterised in that** it comprises a fixation substrate in order to permit its application to surfaces.

9. Construction element **characterised in that** it comprises a substrate of the block or plate type in which the photocatalytic mixture forms part of its composition according to any of claims 1 to 7.

10. Construction element **characterised in that** it includes a substrate of the block or plate type in which at least one of its faces comprises a layer or an impregnation of a photocatalytic mixture according to any of claims 1 to 7.

11. Use of a mixture according to claims 1 to 8 **characterised in that** said mixture is applied on a surface exposed to a gaseous environment containing nitrogen oxides; and the surface on which the mixture is applied is exposed to UV radiation coming from sunlight or artificial light.

12. Procedure for obtaining a mixture according to claims 1 and 2 **characterised in that** a process is carried out in which TiO₂ powder and feldspartoid material powder are mixed, following which they are milled until the same granulometry has been obtained for the two components.

13. Procedure for obtaining a mixture according to claim 12 **characterised in that** the milling process is carried out using a microsphere mill.

14. Procedure for obtaining a mixture according to claims 1 and 3 **characterised in that** feldspartoid material powder is taken, along with TiO₂ powder with a granulometry of the feldspartoid material greater than that of the TiO₂, and TiO₂ powder is projected onto the feldspartoid material until it is coated; and finally the photocatalytic mixture obtained is left to dry.

15. Procedure according to claim 14 in which the contribution of feldspartoid material is made on the basis of feldspartoid material precursors and/or titanium oxide, producing the phase transformation in the physical, chemical or physical-chemical treatment.

16. Procedure according to claim 15 in which an intimate mixture of zeolite 4A and titanium dioxide is subjected to baking in adequate mass proportion and it is thermally treated at temperatures between 600 and 1200°C.

17. Procedure according to claim 16 in which the temperature of the transformation of the mixture of 4A zeolite and titanium dioxide is preferably in a range of 600°C to 800°C.
